Europäisches Patentamt

European Patent Office

Office européen des brevets

(19)

(11) Numéro de publication: **0 256 956 B1**

# (12) FASCICULE DE BREVET EUROPEEN

(45) Date de publication de fascicule du brevet:
**09.10.91**

(51) Int.Cl.⁵: **A61M 1/16, A61M 1/34**

(21) Numéro de dépôt: **87420204.7**

(22) Date de dépôt: **28.07.87**

(54) **Installation à fonctions multiples pour la suppléance à la filtration naturelle du sang.**

(30) Priorité: **08.08.86 FR 8611663**

(43) Date de publication de la demande:
**24.02.88 Bulletin 88/08**

(45) Mention de la délivrance du brevet:
**09.10.91 Bulletin 91/41**

(84) Etats contractants désignés:
**BE DE ES FR GB IT NL**

(56) Documents cités:
**DE-A- 3 444 671**

**TRANSACTIONS OF THE AMERICAN SOCIETY FOR ARTIFICIAL INTERNAL ORGANS, vol. XXV, 1979, pages 404-408; J.H. MILLER et al.: "A volume controlled apparatus for ultrafiltration and hemofiltration with acetate or bicarbonate solutions"**

**TRANSACTIONS OF THE AMERICAN SOCIETY FOR ARTIFICIAL INTERNAL ORGANS, vol. XXVIII, 1982, pages 24-27, Chicago, Illinois, US; M. USUDA et al.: "New simultaneous HF and HD with no infusion fluid"**

(73) Titulaire: **HOSPAL INDUSTRIE**
**7, Avenue Lionel Terray**
**F-69330 Meyzieu(FR)**

(72) Inventeur: **Richalley, Gérard**
**68, rue Duquesne**
**F-69006 Lyon(FR)**
Inventeur: **Delaunay, Marc**
**26, Avenue Sylvin**
**F-69150 Decines(FR)**

## Description

La présente invention entre dans le domaine technique de l'insuffisance rénale et elle concerne les matériels de suppléance mis en oeuvre pour filtrer, extracorporellement, le sang, dans les cas de déficiences aigües temporaires ou de déficiences chroniques.

Pour assumer cette fonction de suppléance, la technique antérieure est riche de plusieurs méthodes permettant d'établir, en outre, un contrôle de leur déroulement.

Une première méthode consiste à mettre en oeuvre un échangeur, c'est-à-dire un appareil divisé en deux compartiments par une membrane semi-perméable. L'un des compartiments est relié au patient par des canalisations de circulation extracorporelle du sang, tandis que le second compartiment est relié à une canalisation de circulation de l'ultrafiltrat extrait du sang qui est récolté dans une poche collectrice réservée à cet effet.

Une telle installation peut faire intervenir une circulation spontanée ou assistée du sang. Le principe de fonctionnement d'une telle installation est basé sur un phénomène de convexion par différence de pression permettant d'extraire et d'éliminer l'eau en excès du sang.

Cette méthode est connue sous la dénomination d'ultrafiltration continue. Elle est particulièrement adaptée à l'élimination rapide de l'excédent d'eau et permet, simultanément, une élimination limitée des déchets du métabolisme drainés par l'ultrafiltrat extrait.

Une telle méthode convient particulièrement bien au traitement des cas aigus pour lesquels, la première préoccupation, est le retour du patient à un poids acceptable par élimination de l'eau en excès.

Une telle méthode n'est, par contre, pas convenablement adaptée à l'élimination des déchets du métabolisme. L'épuration du sang est en effet limitée par la quantité d'ultrafiltrat que l'on peut extraire du sang.

Une autre méthode, dénommée hémofiltration continue, consiste à mettre en oeuvre une installation identique à celle décrite ci-dessus, mais qui est, toutefois, complétée par le raccordement, à la canalisation de circulation extracorporelle du sang, d'une canalisation d'infusion spontanée ou assistée d'une solution physiologique stérile et apyrogène qui est donc ajoutée au sang pour compenser à la perte de poids près, la quantité d'ultrafiltrat extrait.

Il s'agit, encore, d'une méthode qui utilise le phénomène de convexion faisant intervenir une différence de pression, mais l'apport de la solution physiologique permet de réaliser, en prévoyant des temps de traitement plus longs, une extraction sensiblement plus importante des déchets du métabolisme.

En raison de l'infusion d'une solution physiologique, on comprend qu'une telle méthode peut ne pas être appropriée dans la phase initiale du traitement pour certains cas aigus dans lesquels l'objectif prioritaire est le retour rapide à un poids normal du patient. C'est la raison pour laquelle, en pratique, il est fréquent de substituer une telle méthode à la première après que celle-ci ait permis d'atteindre l'objectif prioritaire et qu'il convient alors d'assurer réellement l'élimination des déchets du métabolisme en excès.

Une troisième méthode, connue sous la dénomination hémodialyse continue, consiste à raccorder l'entrée du second compartiment de l'échangeur de l'installation ci-dessus à un réservoir d'une solution physiologique qui est amenée à circuler dans le second compartiment, généralement à contre-courant de la circulation du sang, en respectant une relation de pression et une relation de débit par rapport aux conditions de circulation du sang.

La relation principale de débit est établie de manière à créer des conditions de diffusion à travers la membrane semi-perméable dans le sens compartiment sanguin-compartiment liquide physiologique. D'autre part, la relation de pression a pour effet de créer un transfert par convexion du compartiment sanguin vers le compartiment liquide physiologique. Par ce moyen, il devient possible d'améliorer, par la circulation du liquide physiologique, l'extraction des déchets du métabolisme et, plus particulièrement, l'élimination des petites molécules telles que l'urée.

Cette méthode présente ainsi une possibilité de traitement plus efficace car elle permet une meilleure épuration du sang, selon les critères communément admis.

Il peut donc être considéré que les trois méthodes connues en soi, dans la succession selon le rappel ci-dessus, présentent une efficacité décroissante pour l'élimination de l'eau en excès et une efficacité croissante pour l'élimination des déchets du métabolisme.

Cliniquement, il est fréquent de constater, pour les cas aigus des évolutions de comportement de l'organisme déficient en cours de traitement, nécessitant le recours à une méthode de traitement différente de celle en cours d'application.

Ainsi, après une phase de traitement par une méthode d'ultrafiltration continue, en vue de rétablir un poids normal, il est fréquent de recourir à la méthode d'hémofiltration continue ou d'hémodialyse continue. Cependant, l'application de l'une ou l'autre des méthodes permet, parfois, de constater une nouvelle rétention d'eau devant être de nouveau éliminée rapidement par le recours à la méthode première.

D'autre part, pendant une phase de traitement par hémofiltration continue, il est possible de constater des modifications du catabolisme du patient nécessitant une épuration plus efficace du sang qui ne peut, dans certains cas, être réalisée que par l'application de la méthode d'hémodialyse continue.

Les praticiens sont donc confrontés aux problèmes de changements fréquents d'installations de mise en oeuvre de traitement, de façon à répondre à l'évolution du cas clinique en cours de traitement.

A l'heure actuelle, le recours à l'une ou l'autre des méthodes implique, obligatoirement, de faire intervenir une installation propre à la méthode considérée et de réaliser, à chaque fois, le branchement d'une telle installation, en relation, par les moyens connus appropriés, avec le patient à traiter. Une telle façon de procéder n'est pas satisfaisante, car elle représente une sujétion importante pour le personnel chargé du suivi du cas clinique à traiter, une source d'erreurs non négligeable dans la prise en compte des branchements et/ou des relations de pression et de débit et implique de disposer d'un nombre d'installations important en réserve, étant donné que, par principe, chaque installation ayant fait l'objet d'un branchement spécifique ne peut être modifiée sans prendre de risque d'infection.

Il s'avère donc l'existence certaine d'un besoin pour les praticiens, de pouvoir disposer d'un moyen leur permettant de faire intervenir, en cours de traitement, avec possibilité de commutation, l'une des trois méthodes, selon qu'il convient de prendre en compte, en tant qu'objectif prioritaire, plutôt une perte de poids qu'une élimination de déchets moyenne ou élevée, ou inversement.

Des tentatives, dans le domaine de la dialyse chronique, ont déjà été faites pour proposer des installations permettant d'effectuer le traitement approprié, au choix du praticien.

Ainsi, l'article intitulé "A volume controlled apparatus for ultrafiltration and hemofiltration with acetate or bicarbonate solution" publié au Vol XXV de "Transactions of the American Society for Artificial Internal Organs" de 1979, décrit une installation très complexe, permettant d'effectuer plusieurs thérapies ; cet appareil qui est défini comme un appareil de contrôle d'écoulement des fluides est une installation lourde, inadaptée au traitement des malades aigus des services de réanimation ainsi qu'aux malades des pays dont les équipements médicaux sont limités et qui ne disposent pas du personnel médical adéquat. Une telle installation n'est en outre pas adaptée à une circulation spontanée du sang et/ou liquide de dialyse.

C'est justement l'objectif de la présente invention que de proposer une nouvelle installation conçue pour permettre la mise en oeuvre, de façon commutable, de l'une quelconque des trois méthodes de suppléance, à partir des mêmes moyens techniques, en ne faisant intervenir, pour le personnel chargé de la surveillance et de la conduite du traitement, qu'une seule manoeuvre dans le cas de configuration le plus simple, pour passer de l'une à une autre méthode.

L'objet de l'invention est donc de proposer une nouvelle installation dont le raccordement à un patient permette le recours à l'une quelconque des méthodes de traitement avec possibilité de commutation selon l'objectif prioritaire qu'il convient d'atteindre.

Un autre objet de l'invention est de proposer une nouvelle installation, à caractère simple, fiable dans le temps et peu onéreux, permettant, en conséquence, de maintenir un coût économique acceptable, tout en préservant l'usage unique qu'il convient de réserver à une telle installation.

Un autre objet de l'invention est de proposer une nouvelle installation qui offre la possibilité de faire intervenir, le cas échéant et selon le souhait du praticien, une quatrième méthode de traitement consistant en l'application simultanée d'une méthode d'hémofiltration continue et d'hémodialyse continue, appelée hémodiafiltration continue.

Un autre objet de l'invention est de proposer une nouvelle installation qui puisse être, malgré son caractère à usage unique, aisément adaptée à l'établissement d'une circulation sanguine extracorporelle spontanée ou assistée et/ou d'une solution physiologique spontanée par gravité ou forcée et/ou une extraction de l'ultrafiltrat et/ou du liquide de dialyse également spontanée ou assistée.

Pour atteindre les objectifs cités ci-dessus, l'installation à fonctions commutables pour la suppléance à la filtration naturelle du sang, du type comprenant :

- un échangeur définissant, de part et d'autre d'une membrane semi-perméable, un premier compartiment réservé à la circulation du sang et un second compartiment réservé à la circulation d'un liquide de dialyse et/ou d'un ultrafiltrat,
- des canalisations reliant l'entrée et la sortie dudit premier compartiment au patient,
- une source d'une solution physiologique stérile,
- deux canalisations aptes à conduire ladite solution physiologique, respectivement à l'entrée dudit second compartiment dudit échangeur et à l'une desdites canalisations reliant ledit premier compartiment dudit échangeur au patient,

est caractérisée en ce qu'elle comprend en outre :

- des moyens de réception dudit liquide de dialyse et/ou dudit ultrafiltrat,

- une canalisation reliant la sortie dudit second compartiment aux moyens de réception,
- une vanne trois voies placée à la sortie de ladite source,
- une résistance hydraulique apte à agir sur les conditions de circulation de la solution physiologique,

et en ce que la circulation du sang ainsi que la circulation du liquide de dialyse et/ou de l'ultrafiltrat peuvent être spontanées ou assistées.

Diverses autres caractéristiques ressortent de la description faite ci-dessous en référence aux dessins annexés qui montrent à titre d'exemples non limitatifs, des formes de réalisation de l'objet de l'invention.

La figure 1 représente, de façon schématique, un premier mode de réalisation de la présente invention.

La figure 2 représente, de façon schématique, l'objet de la présente invention fonctionnant selon la méthode d'hémofiltration continue.

La figure 3 représente, de façon schématique, l'objet de la présente invention fonctionnant selon la méthode d'hémodialyse continue.

La figure 4 représente, de façon schématique, un second mode de réalisation de la présente invention.

La figure 5 représente de façon schématique un troisième mode de réalisation de la présente invention.

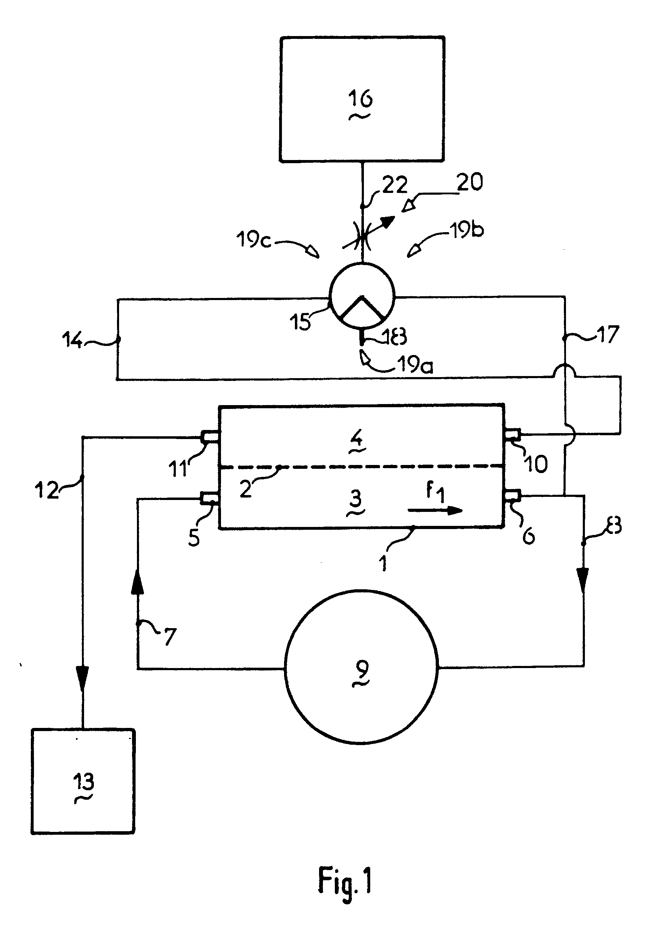

La figure 1 montre, de façon schématique, une installation à fonctions commutables pour la suppléance à la filtration naturelle du sang, conforme à l'invention. Cette installation comprend un échangeur 1 dont les caractéristiques sont choisies pour permettre la mise en oeuvre indifférente de l'une ou l'autre des trois méthodes de traitement connues. Cet échangeur doit avoir à la fois les qualités d'un hémodialyseur et celles d'un hémofiltre. L'échangeur BIOSPAL SCU-CAVH vendu par la société HOSPAL convient bien. L'échangeur 1 comprend, intérieurement, une membrane 2, délimitant deux compartiments de circulation, respectivement 3 et 4.

Le compartiment 3, réservé à la circulation sanguine, possède une tubulure d'entrée 5 et une tubulure de sortie 6 permettant le raccordement par des canalisations 7 et 8 à un patient 9.

Le compartiment 4 possède deux tubulures, respectivement d'entrée 10 et de sortie 11. La tubulure 11 est raccordée par une canalisation 12 à une poche 13 de réception et de collecte du liquide de dialyse et/ou de l'ultrafiltrat.

La tubulure 10 est raccordée à une canalisation 14 qui est issue d'une des sorties d'une vanne trois voies 15 reliée à la sortie d'un réservoir 16 contenant une solution physiologique stérile et de préférence apyrogène par une canalisation 22. La seconde sortie de la vanne trois voies 15 est prolongée par une canalisation 17 qui est branchée sur la canalisation 8 de sortie ou éventuellement la canalisation 7 d'entrée du compartiment sang 3 de l'échangeur 1. La vanne trois voies 15 possède un index 18 susceptible d'être placé face à l'un quelconque de trois repères 19a, 19b, et 19c, associés à des informations en langage clair ou symbolisé correspondant, pour le personnel chargé de la mise en oeuvre et de la surveillance d'une telle installation, à l'une des trois méthodes de suppléance connues. Le repère 19a peut, par exemple, correspondre à la mise en oeuvre d'une méthode d'ultrafiltration continue, le repère 19b à une méthode d'hémofiltration continue et le repère 19c à une méthode d'hémodialyse continue.

L'installation est, par ailleurs, complétée par une résistance hydraulique 20 telle par exemple un clamp réglable ou un robinet de réglage du débit qui est conçu de manière à pouvoir agir sur les conditions de circulation de la solution physiologique.

L'installation décrite ci-dessus fonctionne de la façon suivante.

Dans l'état selon la fig. 1, la vanne trois voies 15 est réglée, en relation avec le repère 19a et ferme les entrées des canalisations 14 et 17.

Lorsque la circulation sanguine extracorporelle est établie dans le sens des flèches, le sang du patient 9 circule à l'intérieur du compartiment 3, dans le sens de la flèche f1. Ceci permet de réaliser, par convexion à travers la membrane 2, une élimination de l'eau en excès qui est évacuée par la canalisation 12, en direction de la poche collectrice 13.

L'installation selon l'invention permet ainsi l'établissement d'une méthode de traitement par ultrafiltration continue.

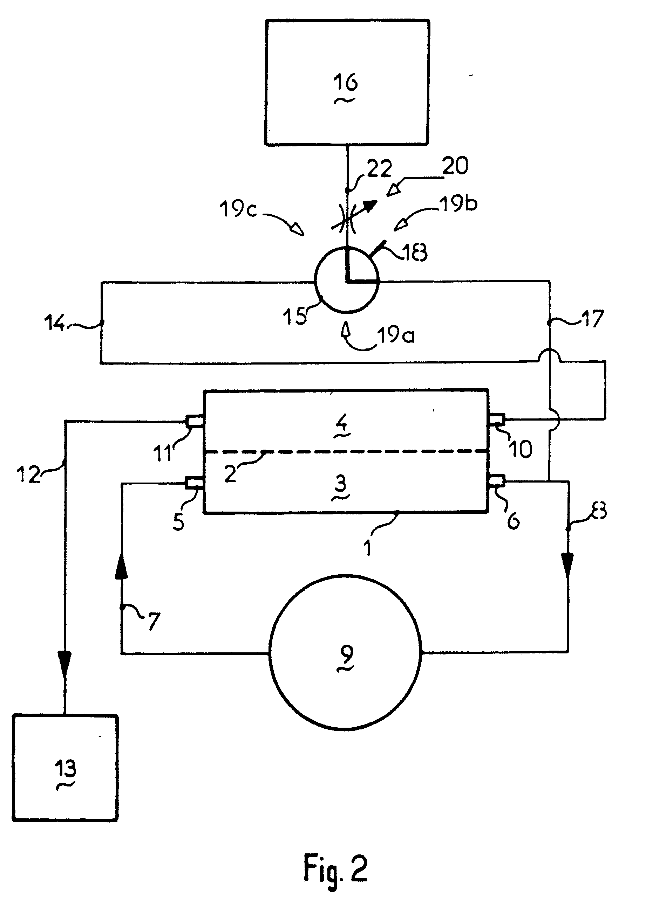

Lorsque la vanne trois voies 15 est manoeuvrée pour placer l'index 18 en regard du repère 19b (fig 2), la canalisation 17 est mise en relation avec le réservoir 16. Lorsque ce réservoir est placé en charge par rapport à l'organisme 9, une circulation par gravité s'établit et la solution physiologique contenue dans le réservoir 16 traverse la vanne trois voies 15 pour emprunter la canalisation 17 et être infusée dans la canalisation 8. On réalise ainsi une post-dilution du sang. On peut aussi, si désiré, introduire le liquide physiologique dans la canalisation 7, à l'entrée de l'échangeur 1, ce qui constitue une pré-dilution du sang.

La résistance hydraulique 20 permet de faire varier le débit de la solution physiologique stérile, la pression provenant de la hauteur du réservoir 16 étant à une valeur compatible avec celle du sang, de manière à permettre l'infusion de la solution physiologique dans le circuit de circulation extracorporelle du sang.

Il s'agit dans ce cas de l'application de la méthode d'hémofiltration, étant donné qu'une charge de solution physiologique est astreinte à emprunter le circuit de circulation extracorporelle du sang, pour favoriser l'élimination des déchets qui sont prélevés par convexion lors du passage du sang à l'intérieur du compartiment 3.

Outre la vérification d'une hauteur de poche convenable, le passage de la première à la seconde méthode s'est donc réalisé uniquement par une intervention sur la vanne trois voies 15 manoeuvrée sans risque d'erreur, de façon que son index 18 soit placé en relation avec la position prédéterminée correspondant à la nouvelle méthode recherchée.

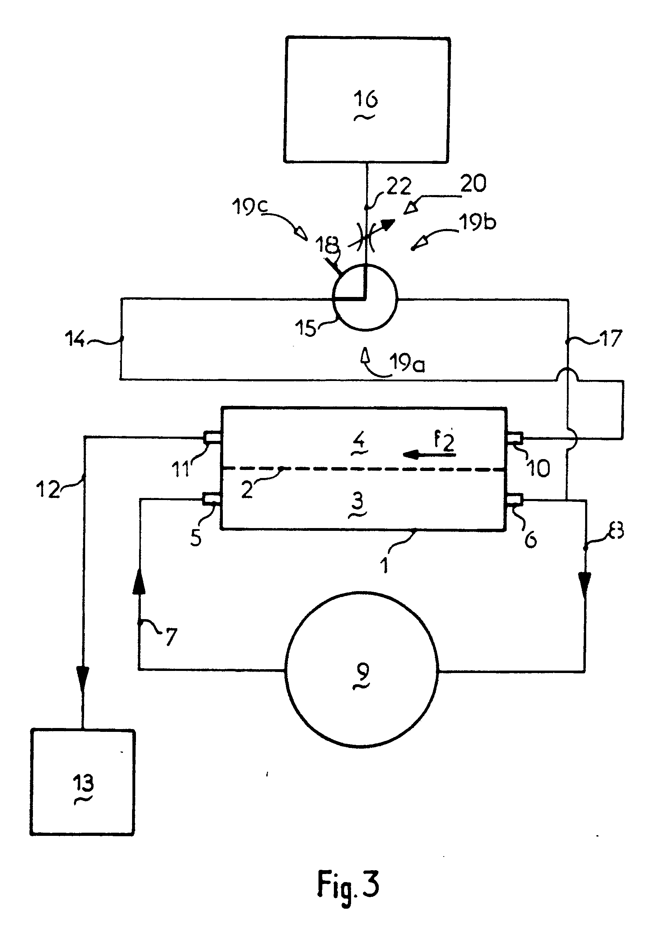

Lorsque la vanne trois voies 15 est manoeuvrée de manière que son index 18 soit placé en relation avec le repère 19c (fig. 3), la solution physiologique du réservoir 16 traverse la résistance hydraulique 20 en empruntant la canalisation 14. Cette solution est ainsi amenée à traverser le compartiment 4, dans le sens de la flèche f2 et à réaliser, de la sorte, par diffusion, l'hémodialyse continue du sang circulant dans le compartiment 3.

La résistance hydraulique 20 permet de régler le débit de la solution physiologique de manière à créer des conditions de diffusion entre le sang et la solution physiologique stérile. La hauteur de charge du réservoir 16 de solution physiologique stérile est ajustée de manière à permettre la circulation de la solution physiologique stérile dans le compartiment (4) de l'échangeur sans entraver la circulation du sang dans l'autre compartiment (3).

Ainsi que cela ressort de la comparaison des fig. 1 à 3, la manoeuvre simple et indubitable de la vanne à trois voies 15 permet de faire intervenir, à partir d'une même installation, l'application d'une méthode d'ultrafiltration continue, d'hémofiltration continue ou d'hémodialyse continue, sans aucun risque d'erreur, de fausse interprétation, de débranchement ou de rebranchement de l'installation

Par des moyens simples, il devient ainsi possible de répondre à l'objectif visé qui est de pouvoir offrir à tout praticien la possibilité de recourir, à partir d'une même installation, à la mise en oeuvre d'une quelconque des méthodes de suppléance en fonction de l'évolution du traitement qu'il constate.

L'installation décrite ci-dessus peut être réalisée comme chacune des installations connues, de façon simple et peu onéreuse, en considération de l'objectif d'usage unique qui lui est appliqué, étant donné qu'il est possible de choisir, dans la technique connue, une vanne trois voies 15 et une résistance hydraulique 20 de faible coût, aptes à assumer les fonctions qui leur sont dévolues de façon fiable. Bien que la première fonction assurée par la vanne trois voies 15 soit la sélectivité de circulation de la solution physiologique stérile, elle doit également présenter une excellente étanchéité.

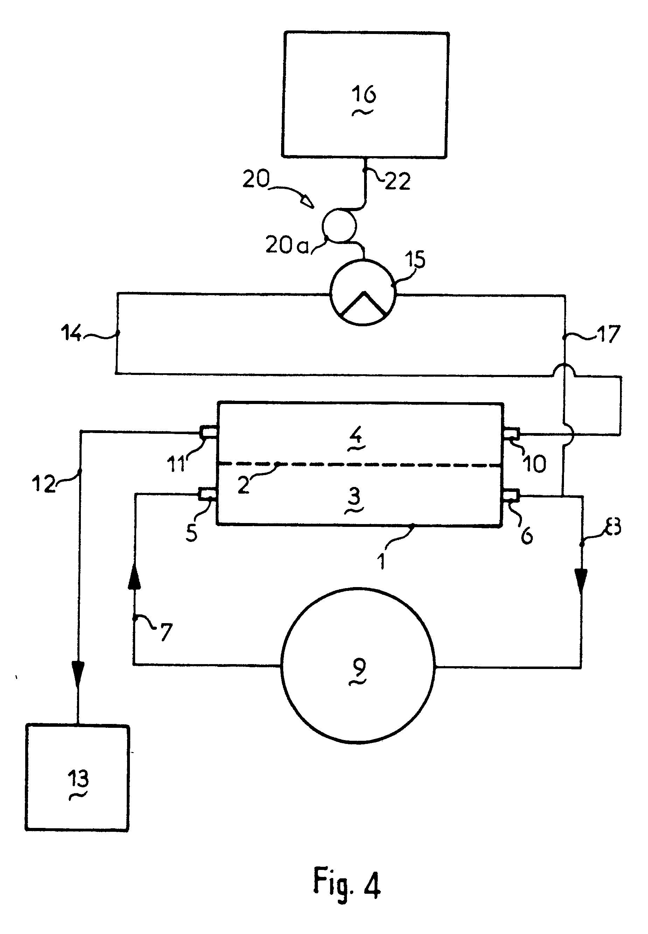

La fig 4 montre qu'il peut être prévu d'utiliser, de façon avantageuse, non pas une résistance hydraulique à caractère statique, comme dans l'exemple selon la fig. 1, mais une résistance hydraulique à caractère dynamique, par exemple constituée par une pompe 20a dont les conditions de fonctionnement peuvent être réglées par l'opérateur pour fournir une solution physiologique dont les conditions de circulation sont compatibles avec l'établissement d'une méthode de traitement par hémofiltration continue ou par hémodialyse continue.

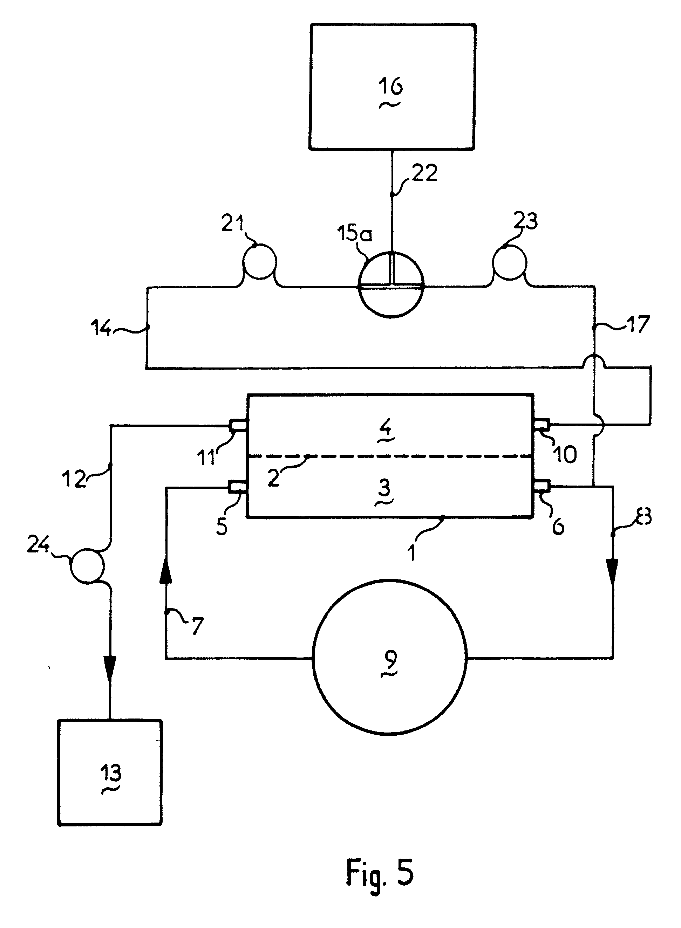

Dans le cas, sel la figure 5, où on désire réaliser simultanément une hémodialyse et une hémofiltration continues (technique appelée également hémodiafiltration continue), on peut utiliser avantageusement un robinet trois voies 15a dont le boisseau est muni d'une canalisation en T permettant de relier le réservoir 16 simultanément à la canalisation 14 de circulation du liquide physiologique servant de liquide de dialyse et à la canalisation 17 d'infusion du liquide physiologique dans le circuit extracorporel de sang.

La figure 5 montre que l'installation peut comporter outre la pompe 20a, (non représentée) une pompe 24 pour assister l'extraction du liquide de dialyse et de l'ultrafiltrat.

On peut, de plus régler les conditions de circulation de la solution physiologique dans les canalisations 14 et 17 par des clamps, ou mieux par des pompes 21 et 23.

Les pompes 20a, 21, 23 et 24 sont, de préférence, du type péristaltique.

L'invention n'est pas limitée aux exemples décrits et représentés car diverses modifications peuvent y être apportées sans sortir de son cadre, tel que de'fini par les revendications suivantes.

## Revendications

1. Installation à fonctions commutables pour la suppléance à la filtration naturelle du sang, du type comprenant un échangeur (1) définissant, de part et d'autre d'une membrane semi-perméable (2), un premier compartiment (3) réservé à la circulation du sang et un second compartiment (4) réservé à la circulation d'un liquide de dialyse et/ou d'un ultrafiltrat et des canalisations (7,8) reliant l'entrée et la sortie dudit premier compartiment (3) au patient (9), une source (16) d'une solution physiologique stérile, deux canalisations (14,17) aptes à conduire ladite solution physiologique, respectivement à l'entrée dudit second compartiment (4) dudit échangeur (1) et à l'une desdites canalisations (7,8) reliant ledit premier compartiment (3) dudit échangeur (1) au patient (9), caractérisée en ce qu'elle comprend en outre :

- des moyens (13) de réception dudit liquide de dialyse et/ou dudit ultrafiltrat,
- une canalisation (12) reliant la sortie dudit second compartiment aux moyens (13) de réception,
- une vanne trois voies (15) placée à la sortie de ladite source (16),
- une résistance hydraulique (20) apte à agir sur les conditions de circulation de la solution physiologique, et en ce que la circulation du sang ainsi que la circulation du liquide de dialyse et/ou de l'ultrafiltrat peuvent être spontanées ou assistées.

2. Installation selon la revendication 1, caractérisée en ce que la résistance hydraulique (20) est du type statique.

3. Installation selon la revendication 1, caractérisée en ce que la résistance hydraulique (20) est du type dynamique.

4. Installation selon l'une des revendication 1 à 3, caractérisée en ce que la résistance hydraulique (20) est disposée en amont de la vanne (15) par rapport au réservoir(16).

5. Installation selon l'une des revendications 1 à 4, caractérisée en ce qu'elle comprend, en outre, une pompe (24) placée sur la canalisation (12) d'extraction du liquide de dialyse et/ou de l'ultrafiltrat.

6. Installation selon l'une des revendications 1 à 5 caractérisée en ce qu'elle comprend, en outre, une pompe (23) placée sur la canalisation (17) issue de la vanne et branchée sur l'une ou l'autre des canalisations (7,8) de circulation extracorporelle du sang.

7. Installation selon l'une des revendications 1 à 6 caractérisé en ce qu'elle comprend, en outre, une pompe (21) placée sur la canalisation (14) issue de la vanne et branchée à l'entrée du second compartiment (4) de l'échangeur (1).

8. Installation selon l'une des revendications 1 et 3 à 7 caractérisée en ce que la résistance hydraulique est une pompe (20 a).

**Claims**

1. Installation with interchangeable functions for the replacement of the natural filtration of the blood of the type comprising an exchanger (1) defining on either side of a semipermeable membrane (2), a first compartment (3) reserved for blood circulation and a second compartment (4) reserved for circulation of a dialysis liquid and/or of an ultrafiltrate and lines (7,8) connecting the inlet and the outlet of the said first compartment (3) to the patient (9), a source (16) of a sterile physiological solution, two lines (14, 17) capable of conveying the said physiological solution repectively to the inlet of the said second compartment (4) of the said exchanger (1) and to one of the said lines (7, 8) connecting the said first compartment (3) of the said exchanger (1) to the patient (9), characterised in that it moreover comprises :

- receiving means (13) for the said dialysis liquid and/or the said ultrafiltrate,
- a line (12) connecting the outlet of the said second compartment to the receiving means (13),
- a three way valve (15) placed at the outlet of the said source (16),
- a hydraulic resistance (20) capable of acting on the circulation conditions of the physiological solution, and in that the blood circulation as well as the circulation of the dialysis liquid and/or of the ultrafiltrate can be spontaneous or assisted.

2. An installation according to claim 1 characterised in that the hydraulic resistance (20) is of the static type.

3. An installation according to claim 1 characterised in that the hydraulic resistance (20) is of the dynamic type.

4. An installation according to one of claim 1 to 3, characterised in that the hydraulic resistance (20) is disposed ahead of the valve (15) in relation to the reservoir (16).

5. An installation according to one of claims 1 to 4, characterised in that it comprises, moreover, a pump (24) placed on the line (12) for the extraction of the dialysis liquid and/or of the ultrafiltrate.

6. An installation according to one of claims 1 to 5 characterised in that it comprises, moreover, a pump (23) placed on the line (17) coming from the valve and connected to one or the other of the lines (7, 8) for the extracorporeal blood circulation.

7. An installation according to one of claims 1 to 6, characterised in that it comprises, moreover, a pump (21) placed on the line (14) coming from the valve and connected to the inlet of the second compartment (4) of the exchanger (1).

8. An installation according to one of claims 1 and 3 to 7, characterised in that the hydraulic resistance is a pump (20a).

**Patentansprüche**

1. Vorrichtung mit Schaltfunktion für den Ersatz der natürlichen Blutfiltlierung vom Typ umfassend einen Austauscher (1), der beiderseits einer semipermeablen Membran (2) eine erste Kammer (3), die für den Blutkreislauf vorgesehen ist, und eine zweite Kammer (4) aufweist, die für den Kreislauf einer Dialyseflüssigkeit und/oder eines Ultrafiltrats vorgesehen ist, Leitungen (7,8), die den Einlaß und den Auslaß der ersten Kammer (3) mit dem Patienten (9) verbinden, eine Quelle (16) einer physiologischen, sterilen Lösung und zwei Leitungen (14,17), die geeignet sind zum Leiten der physiologischen Lösung, jeweils am Einlaß der zweiten Kammer (4) des Austauschers (1) und an einer der Leitungen (7,8), die die erste Kammer (3) des Austauschers (1) mit dem Patienten (9) verbindet,
   **dadurch gekennzeichnet,**
   daß sie außerdem umfaßt:
   - Einrichtungen (13) zur Aufnahme der Dialyseflüssigkeit und/oder des Ultrafiltrats,
   - eine Leitung (12), die den Auslaß der zweiten Kammer mit den Aufnahmeeinrichtungen (13) verbindet,
   - ein Dreiwegeventil (15), das am Auslaß der Quelle (16) angeordnet ist,
   - einen hydraulischen Widerstand (20), der geeignet ist, auf die Bedingungen des Kreislaufs der physiologischen Lösung einzuwirken, und
   daß der Blutkreislauf sowie der Kreislauf der Dialyseflüssigkeit und/oder des Ultrafiltrats selbsttätig oder unterstützt sein können.

2. Vorrichtung nach Anspruch 1,
   **dadurch gekennzeichnet,**
   daß der hydraulische Widerstand (20) vom statischen Typ ist.

3. Vorrichtung nach Anspruch 1,
   **dadurch gekennzeichnet,**
   daß der hydraulische Widerstand (20) vom dynamischen Typ ist.

4. Vorrichtung nach den Ansprüchen 1 bis 3,
   **dadurch gekennzeichnet,**
   daß der hydraulische Widerstand (20) stromaufwärts des Dreiwegeventils (15) bezüglich der Quelle (16) angeordnet ist.

5. Vorrichtung nach einem der Ansprüche 1 bis 4,
   **dadurch gekennzeichnet,**
   daß sie außerdem eine Pumpe (24) umfaßt, die in der Leitung (12) zur Extraktion der Dialyseflüssigkeit und/oder des Ultrafiltrats angeordnet ist.

6. Vorrichtung nach einem der Ansprüche 1 bis 5,
   **dadurch gekennzeichnet,**
   daß sie außerdem eine Pumpe (23) umfaßt, die in der Leitung (17) angeordnet ist, die am Ventil beginnt und mit der einen oder der anderen der Leitungen (7,8) des außerkörperlichen Blutkreislaufes verbunden ist.

7. Vorrichtung nach einem der Ansprüche 1 bis 6,
   **dadurch gekennzeichnet,**
   daß sie außerdem eine Pumpe (21) umfaßt, die in der Leitung (14) angeordnet ist, die am Ventil beginnt und mit dem Einlaß der zweiten Kammer (4) des Austauschers (1) verbunden ist.

8. Vorrichtung nach einem der Ansprüche 1 und 3 bis 7,
   **dadurch gekennzeichnet,**
   daß der hydraulische Widerstand eine Pumpe (20a) ist.

Fig.1

Fig. 2

**Fig. 3**

Fig. 4

Fig. 5